(19) Europäisches Patentamt
European Patent Office
Office européen des brevets

(11) Veröffentlichungsnummer: **0 003 788**
**B1**

(12) # EUROPÄISCHE PATENTSCHRIFT

(45) Veröffentlichungstag der Patentschrift: **09.12.81**

(51) Int. Cl.³: **C 07 H 15/04,** C 07 H 15/18, C 07 H 13/12 // A61K31/70

(21) Anmeldenummer: **79100400.5**

(22) Anmeldetag: **12.02.79**

(54) **Glucofuranosyl-nitrosoharnstoffderivate, Verfahren zu deren Herstellung und ihre pharmazeutischen Präparate.**

(30) Priorität: **21.02.78 CH 1849/78**

(43) Veröffentlichungstag der Anmeldung: **05.09.79 Patentblatt 79/18**

(45) Bekanntmachung des Hinweises auf die Patenterteilung: **09.12.81 Patentblatt 81/49**

(84) Benannte Vertragsstaaten: **BE CH DE FR GB IT LU NL SE**

(73) Patentinhaber: **CIBA-GEIGY AG, Patentabteilung Postfach, CH-4002 Basel (CH)**

(72) Erfinder: **Stanek, Jaroslav, Dr., Florastrasse 6, CH-4127 Birsfelden (CH)**

(56) Entgegenhaltungen:
**EP-A-0 000 126**
**CHEMICAL ABSTRACTS, Vol. 86, Nr. 3, 17. Januar 1977, *American Chemical Society*, Nr. 1 6911j, Columbus, Ohio U.S.A.**
**G. KIMURA et al.: «5-(Nitrosoureido)-5-deoxy-D-ribofuranose derivatives», Seite 451**

**CHEMICAL ABSTRACTS, Vol. 88, Nr. 5, 30. Januar 1978, American Chemical Society, Nr. 38087p Columbus, Ohio, U.S.A.**
**J.L. MONTERO et al.: «Glycosylnitrosoureas: *Study of the D-ribose series*» Seite 529**

(56) Entgegenhaltungen:
**CHEMICAL ABSTRACTS, Vol. 88, Nr. 7 13. Februar 1978, American Chemical Society, Nr. 51099p *Columbus, Ohio, U.S.A.***
**J.L. MONTERO et al.: «New synthesis of 1-(3-(2-chloroethyl)-3-nitro eido)-2,3-0-isopropylidene-5-0-p-nitrobenzoyl-D-furanose», Seite 573**

**CHEMICAL ABSTRACTS, Vol. 89, Nr. 1, 1. Juli 1978, American Chemical Society, Nr. 6509g, Columbus, Ohio, U.S.A.**
**G. KIMURA et al.: «Chloroethylnitrosourea derivatives *of alkyl glucopyranosides*», Seite 562**

**EP 0 003 788 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

# Glucofuranosyl-nitrosoharnstoffderivate, Verfahren zu deren Herstellung und ihre pharmazeutischen Präparate

Gegenstand der Erfindung sind neue $N_1$-Glucofuranosid-6-yl-$N_3$-Nitrosoharnstoffe der allgemeinen Formel

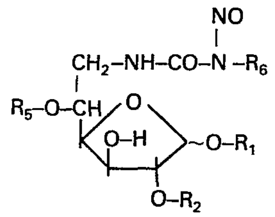

(I)

worin $R_1$, $R_2$ und $R_5$ Wasserstoff, gegebenenfalls durch Hydroxy, Niederalkoxy oder Halogen substituiertes Niederalkyl, gegebenenfalls im aromatischen Teil durch Niederalkyl, Hydroxy, Niederalkoxy, Niederalkylidendioxy, Halogen und/oder Trifluormethyl substituiertes Arylniederalkyl, oder Acyl, $R_1$ und $R_2$ zusammen auch Niederalkyliden oder Cycloalkyliden darstellen, und $R_6$ gegebenenfalls durch Hydroxy, Niederalkoxy oder Halogen substituiertes Niederalkyl bedeutet.

Nachfolgend mit dem Ausdruck «nieder» modifizierte Reste, Radikale oder Verbindungen enthalten, sofern nichts besonderes angegeben, vorzugsweise bis zu 7, in erster Linie bis zu 4 Kohlenstoffatomen.

Niederalkyl ist z.B. Isopropyl, geradkettiges oder verzweigtes, in beliebiger Stellung gebundenes Butyl, Pentyl, Hexyl oder Heptyl und vor allem Methyl, Äthyl oder n-Propyl.

Als Substituenten der gegebenenfalls substituierten Niederalkylgruppe kommen in erster Linie in Betracht freie oder verätherte Hydroxygruppen, z.B. Niederalkoxygruppen, oder Halogenatome. Dabei kann der substituierte Niederalkylrest einen, zwei oder mehrere gleiche oder verschiedene Substituenten, insbesondere freie Hydroxylgruppen oder Halogenatome tragen.

In Arylniederalkyl entspricht der Niederalkylteil vor allem dem obgenannten Niederalkyl und ist in erster Linie Methyl oder Äthyl. Der aromatische Teil ist insbesondere ein mono-cyclischer, sowie bi-cyclischer Arylrest, in erster Linie Phenyl, aber auch Naphthyl. Er kann gegebenenfalls, z.B. durch Niederalkylgruppen, freies oder veräthertes Hydroxy, z.B. Niederalkoxy oder Niederalkylendioxy, Halogenatome und/oder Trifluormethyl mono-, di- oder polysubstituiert sein. Besonders zu nennen sind 2-Phenyläthyl, Chlorbenzyl, Methylbenzyl, Hydroxybenzyl, Methoxybenzyl oder vor allem Benzyl.

Ein Niederalkylidenrest ist etwa der 2-Butyliden-, 3-Pentyliden- und in erster Linie der Isopropylidenrest. Der Cycloalkylidenrest enthält vorzugsweise 5–7 Ringkohlenstoffatome und ist in erster Linie Cyclopentyliden oder Cyclohexyliden.

Acyl ist insbesondere ein Acylrest einer organischen Säure, insbesondere einer organischen Carbonsäure. So ist Acyl insbesondere Alkanoyl, vor allem mit 2–18 Kohl enstoffatomen, wie Acetyl oder Propionyl, oder auch Aroyl, wie Naphthoyl-1, Naphthoyl-2- und insbesondere Benzoyl oder durch Halogen, Niederalkyl, Niederalkoxy, Trifluormethyl, Hydroxy oder Niederalkanoyloxy substituiertes Benzoyl oder Naphthoyl. Acyl kann auch ein Acylrest einer organischen Sulfonsäure sein, z.B. einer Alkansulfonsäure, insbesondere einer Niederalkansulfonsäure, wie Methansulfonsäure oder Äthansulfonsäure, oder einer Arylsulfonsäure, insbesondere einer gegebenenfalls niederalkyl-substituierten Phenylsulfonsäure, wie Benzolsulfonsäure oder p-Toluolsulfonsäure, sowie den Rest einer Carbaminsäure darstellen, wie unsubstituiertes Carbamoyl, Niederalkyl-carbamoyl oder Aryl-carbamoyl, wie Methylcarbamoyl oder Phenyl-carbamoyl.

Niederalkyl, als Substituent der obgenannten Reste ist in erster Linie Methyl oder Äthyl, aber auch n-Propyl, Isopropyl oder geradkettiges oder verzweigtes Butyl.

Niederalkoxy, als Substituent der obgenannten Reste, ist insbesondere Methoxy oder Äthoxy, ferner n-Propoxy, Isopropoxy, n-Butoxy oder Isobutoxy.

Halogen ist z.B. Fluor, Chlor oder Brom.

Die neuen Verbindungen können in der Form von Anomerengemischen oder von reinen α-oder β-Anomeren vorliegen.

Die neuen Verbindungen besitzen wertvolle pharmakologische Eigenschaften, insbesondere zeigen sie eine sehr gute Wirkung bei einigen verschiedenartigen transplantablen Tumoren und Leukämien.

So bewirken sie in Dosen von 25–500 mg/kg i.p. eine starke Hemmung des Tumorwachstums bei Mäusen mit z.B. Ehrlich-Ascites-Karzinom und bei Ratten mit z.B. Walker CaSa 256. Analoge Dosen bewirken eine Lebensverlängerung gegenüber Kontrollen bei Mäusen mit z.B. Leukämie L 1210.

So erreicht man z.B. mit dem Äthyl-6-desoxy-5-0-methyl-6-(3-methyl-3-nitroso-ureido)-β-D-glucofuranosid in Dosen von 50–250 mg/kg i.p. eine 80–100%-ige Hemmung des Wachstums der genannten Tumoren und bei Leukämie L 1210 eine Lebensverlängerung von mehr als 90%. Ähnliche Resultate wurden auch bei po-Applikation erreicht. Die Verträglichkeit ist gut. Nebenwirkungen sind auch bei längerer Behandlung nicht festzustellen. Auch bei normalen Tieren sind nach dreiwöchiger peroraler Behandlung makroskopisch keine Organveränderungen zu sehen.

Zuckerderivate, welche eine Nitrosoharnstoffgruppierung enthalten, sind aus der Literatur bekannt. Solche Verbindungen unterscheiden sich hinsichtlich der strukturellen Gegebenheiten von denen der vorliegenden Anmeldung in erheblichem Masse. Insbesondere weisen z.B. die im Stand der Technik beschriebenen Verbindungen in 3-Stellung eine verätherte Hydroxygruppe auf im Gegensatz zu den die vorliegende Anmeldung betreffenden Substanzen, in denen die in 3-Stellung stehende Hydroxygruppe frei ist.

Die Erfindung betrifft insbesondere Verbindungen der Formel I, worin $R_1$ und $R_2$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl, in erster Linie in para-Stellung, substituiertes Benzyl, $R_1$ und $R_2$ auch Niederalkyliden oder Cycloalkyliden mit 5–6 Kohlenstoffatomen bedeutet, $R_5$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl, in erster Linie in p-Stellung, substituiertes Benzyl, oder Niederalkanoyl, z.B. Acetyl oder Propionyl oder gegebenenfalls durch Halogen, Niederalkoxy, Hydroxy oder Niederalkanoyloxy substituiertes Benzoyl, z.B. p-Chlorbenzoyl p-Brombenzoyl, p-Methoxybenzoyl oder o- oder p-Hydroxybenzoyl und $R_6$ eine gegebenenfalls durch Halogen, Hydroxy oder Niederalkoxy substituiertes Niederalkyl bedeutet.

Hervorzuheben sind insbesondere Verbindungen obiger Formel I, worin $R_1$ Niederalkyl und $R_2$ Wasserstoff oder $R_1$ und $R_2$ zusammen Niederalkyliden bedeuten, $R_5$ Wasserstoff, Niederalkyl oder gegebenenfalls durch Halogen, Hydroxy, Niederalkoxy oder Alkyl, besonders in p-Stellung, substituiertes Benzyl oder $R_6$ gegebenenfalls mit Chlor substituiertes Niederalkyl, z.B. Methyl oder Chloräthyl darstellen.

In erster Linie sind Verbindungen zu nennen, worin $R_5$ Methyl, $R_6$ Methyl oder Chloräthyl und $R_1$ Wasserstoff, Methyl, Äthyl oder Propyl und $R_2$ Wasserstoff oder $R_1$ und $R_2$ zusammen den Isopropylidenrest bedeuten.

Die neuen Verbindungen werden erhalten, wenn man eine Verbindung der Formel II

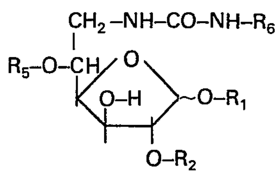

(II)

in an sich bekannter Weise nitrosiert. Dazu setzt man vorzugsweise die Verbindung II mit salpetriger Säure, deren Salzen oder Derivaten um. Vorzugsweise verwendet man dazu ein Salz, wie ein Alkali- oder Erdalkali-, besonders das Natriumsalz der salpetrigen Säure und setzt daraus mit einer Säure, wie einer Mineralsäure, z.B. Salzsäure, Schwefelsäure oder Salpetersäure, einer organischen Säure, wie Kohlensäure, Essigsäure oder einer Sulfonsäure z.B. einer Niederalkansulfonsäure, wie Methan- oder Aethansulfonsäure oder einem Sulfonsäuregruppen enthaltenden Ionenaustauscher, z.B. Amberlite IR 120, die salpetrige Säure frei. Man kann aber auch ein Anhydrid der salpetrigen Säure, insbesondere ein gemischtes Anhydrid mit z.B. der Salpetersäure oder einer Halogenwasserstoffsäure verwenden.

Wenn notwendig, arbeitet man in Gegenwart eines Lösungsmittesl, wobei z.B. eine vorhandene organische Säure ebenfalls als solches verwendet werden kann. Die Reaktion wird vorzugsweise bei tiefer Temperatur z.B. -10 ° bis 30 °C durchgeführt.

Die bei dieser Verfahrensmethode verwendeten Ausgangsstoffe sind neu. Sie lassen sich in an sich bekannter Weise aus einer entsprechenden, in 6-Stellung unsubstituierten Glucofuranose gewinnen, z.B. durch Umsetzen zu einem reaktionsfähigen Ester, z.B. mit einer Alkansulfonsäure, Arylsulfonsäure oder Halogenwasserstoffsäure, dann zu einem Azid und Reduktion des so erhaltenen Azids zur 6-Desoxy-6-amino-glucofuranose, welche dann mit einem geeigneten N-$R_6$-Carbaminsäurederivat, wie einem entsprechenden Isocyanat zur 6-Desoxy-6-(3-$R_6$-ureido)-glucofuranose kondensiert wird. Wie oben erwähnt erfolgen diese Umsetzungen in an sich bekannter Weise.

Eine weitere Methode zur Herstellung der neuen Nitrosoharnstoffe besteht darin, dass man eine Verbindung der Formel III

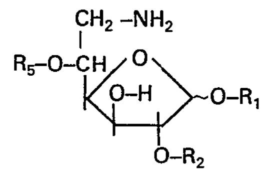

(III)

mit einem reaktionsfähigen Derivat einer

N – Nitroso – N – $R_6$ – carbaminsäure (IV)

umsetzt.

Das reaktionsfähige Derivat kann beispielsweise ein Säureanhydrid, vorzugsweise ein gemischtes Säureanhydrid, wie ein Säureazid oder ein aktivierter Ester sein. Als aktivierte Ester seien insbesondere genannt Cyanmethylester, Carboxymethylester, Paranitrophenylthioester, Paranitrophenylester, 2,4,5-Trichlorphenylester, Pentachlorphenylester, N-Hydroxy-succinimidester, N-Hydroxyphalimidester, 8-Hydroxychinolinester oder N-Hydroxypiperidinester.

Diese Reaktion führt man in an sich bekannter Weise durch, vorzugsweise in einem Lösungsmittel, wie Wasser, einem halogenierten Kohlenwasserstoff, z.B. Dichlor- oder Trichloräthan, einem Aether, wie Diäthyläther, Tetrahydrofuran, Dimethylformamid, Dimethylsufoxyd, oder einem gegebenenfalls alkylierten Pyridin, wie Pyridin, Picolin, Lutidin, oder Chinolin.

Die verwendeten Ausgangsstoffe sind bekannt oder lassen sich in an sich bekannter Weise herstellen. So kann man das Amin der Formel III aus einer entsprechenden, in 6-Stellung unsubstituierten Glucofuranose, z.B. durch Umsetzen zu einem reaktionsfähigen Ester, z.B. mit einer Alkan- oder Arylsulfonsäure oder einer Halogenwasserstoffsäure, und dann zu einem Azid und Reduktion des so erhaltenen Azid zu 6-Desoxy-6-amino-glucofuranose gewinnen.

Die obenbeschriebenen Verfahren werden nach an sich bekannten Methoden durchgeführt, in Abwesenheit oder vorzugsweise in Anwesenheit von Verdünnungs- oder Lösungsmitteln, wenn notwendig, unter Kühlen oder Erwärmen, unter erhöhtem Druck und/oder in einer inerten Atmosphäre, z.B. unter Stickstoff.

Die neuen Verbindungen können als reine α- oder β-Anomere oder als Anomerengemische vorliegen. Letztere können auf Grund der physikalisch-chemischen Unterschiede der Bestandteile in bekannter Weise in die beiden reinen Anomeren aufgetrennt werden, z.B. mittels chromatographischer Trennung, wie Dünnschichtchromatographie oder irgend eines anderen geeigneten Trennverfahrens. Vorzugsweise isoliert man das wirksamere der beiden Anomeren.

Die Erfindung betrifft auch diejenigen Ausführungsformen des Verfahrens bei denen man einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Die vorliegende Erfindung betrifft ebenfalls pharmazeutische Präparate, welche Verbindungen der Formel I enthalten. Bei den erfindungsgemässen pharmazeutischen Präparaten handelt es sich um solche zur enteralen, wie oralen und rektalen, sowie parenteralen Verabreichung an Warmblüter, welche den pharmakologischen Wirkstoff allein oder zusammen mit einem pharmazeutisch anwendbaren Trägermaterial enthalten. Die Dosierung des Wirkstoffes hängt von der Warmblüterspezies, dem Alter und dem individuellen Zustand, sowie von der Applikationsweise ab.

Die neuen pharmazeutischen Präparate enthalten von etwa 10% bis etwa 95%, vorzugsweise von etwa 20% bis etwa 90% des Wirkstoffes. Erfindungsgemässe pharmazeutische Präparate können z.B. in Dosiseinheitsformen wie Dragées, Tabletten, Kapseln, Suppositorien oder Ampullen vorliegen. Die pharmazeutischen Präparate der vorliegenden Erfindung werden in an sich bekannter Weise, z.B. mittels konventioneller Misch-, Granulier-, Dragier-, Lösungs- oder Lyophilisierungsverfahren hergestellt.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder, Polyvinylpyrrolidon, und/oder, wenn erwünscht, Sprengmittel, wie die obgenannten Stärken, ferner Carboxymethylstärke, quervernetztes Polyvinylpyrrolidon, Agar, Alginsäure oder ein Salz davon, wie Natriumalginat, Hilfsmittel sind in erster Linie Fliessregulier- und Schmiermittel, z.B. Kieselsäure, Talk, Stearinsäure oder Salze davon, wie Magnesium- oder Calciumstearat, und/ oder Polyäthylenglykol. Dragée-Kerne werden mit geeigneten, gegebenenfalls Magensaft-resistenten Überzügen versehen, wobei man u.a. konzentrierte Zuckerlösungen, welche gegebenenfalls arabischen Gummi, Talk, Polyvinylpyrrolidon, Polyäthylenglycol und/oder Titandioxid enthalten, Lacklösungen in geeigneten organischen Lösungsmitteln oder Lösungsmittelgemischen oder, zur Herstellung von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder Kennzeichnung verschiedener Wirkstoffdosen, beigefügt werden.

Die Erfindung betrifft ferner die Behandlung von mit Tumoren oder Leukämie behafteter Warmblüter zur Erzielung tumor- und/oder leukämiehemmender Wirkungen durch Verabfolgen eines erfindungsgemässen pharmazeutischen Präparates. Vorteilhaft beträgt dei Tagesdosis bei einem etwa 70 kg schweren Warmblüter etwa 10–500 mg pro Tag, vorzugsweise 50–300 mg pro Tag.

Die nachfolgenden Beispielen illustrieren die oben beschriebene Erfindung; sie sollen jedoch diese in ihrem Umfang in keiner Weise einschränken. Temperaturen werden in Celsiusgraden angegeben.

Beispiel 1

Eine auf 0 ° gekühlte Lösung von 13,5 g Äthyl-6-deoxy-5-0-methyl-6-(3-methylureido)-α-D-glucofuranosid in 150 ml dest. Wasser und 6,2 ml Eisessig wird innerhalb 2 Stunden tropfenweise mit einer Lösung von 3,6 g Natriumnitrit in 20 ml dest. Wasser versetzt. Man rührt das Reaktionsgemisch 16 Stunden im Eisbad, sättigt mit Natriumchlorid und extrahiert fünfmal mit 200 ml Chloroform. Die Chloroformextrakte werden vereinigt, über Natriumsulfat getrocknet und eingedampft. Der Rückstand wird in 100 ml dest. Wasser aufgenommen und gefriergetrocknet. Man erhält das Äthyl-6-deoxy-5-0-methyl-6-(3-methyl-3-nitrosoureido)-α-D-glucofuranosid vom Smp. 54–55 ° C und $[\alpha]_D^{20} = +65° \pm 1°$ (Chloroform, c = 1,003).

Das Ausgangsmaterial kann wie folgt hergestellt werden:

Eine Lösung von 52,6 g 6-Azido-3-0-benzyl-6-deoxy-1,2-0-isopropyliden-α-D-glucofuranose in 600 ml N,N-Dimethylformamid wird mit 8 g Natriumhydrid pract. (oelfrei) versetzt, eine Stunde bei Raumtemperatur gerührt und auf 0 ° C gekühlt. Nun werden während 30 Minuten 19,2 ml Methyljodid zugetropft und das Gemisch eine weitere Stunde gerührt. Man filtriert und dampft die Lösung ein. Der Rückstand wird mit dest. Wasser versetzt und mit Äther extrahiert. Die Ätherlösung wird mit dest. Wasser neutral gewaschen, über Magnesiumsulfat getrocknet und eingedampft. Man erhält so die 6-Azido-3-0-benzyl-6-deoxy-1,2-0-isopropyliden-5-0-methyl-α-D-glucofuranose als gelbliches Oel vom $[\alpha]_D^{20} = -59° \pm 1°$ (Chloroform, c = 0,667).

Eine Lösung von 54,6 g 6-Azido-3-0-benzyl-6-deoxy-1,2-0-isopropyliden-5-0-methyl-α-D-glucofuranose in 430 ml abs. Äthanol und 120 ml 4,6 N alkoholischer Salzsäure wird 18 Stunden bei Raumtemperatur stehen gelassen und im Wasserstrahlvakuum eingedampft. Der Rückstand wird in

Äther aufgenommen, mit einer gesättigten Natriumhydrogencarbonatlösung und dest. Wasser gewaschen, über Magnesiumsulfat getrocknet und eingedampft. Durch eine Säulenchromatographie auf 1,2 kg Kieselgel mit Methylenchlorid/Essigester 19/1 wird das Äthyl-6-azido-3-0-benzyl-6-deoxy-5-0-methyl-α-D-glucofuranosid als farbloses Oel vom $[\alpha]_D^{20} = +35° \pm 1°$ (Chloroform, c = 0,834) erhalten.

10,1 g Äthyl-6-azido-3-0-benzyl-6-deoxy-5-0-methyl-α-D-glucofuranosid in 100 ml Äthanol werden in Gegenwart von 0,5 g 5 % Palladium/Kohle mit Wasserstoff während 90 Minuten reduziert. Nach dem Abfiltrieren des Katalysators wird das Filtrat unter Rühren und Aussenkühlung tropfenweise mit 36,7 ml Methylisocyanat versetzt und zur Trockene eingedampft. Der Rückstand wird aus Essigester/Äther kristallisiert. Man erhält so das Äthyl-3-0-benzyl-6-deoxy-5-0-methyl-6-(3-methylureido)-α-D-glucofuranosid vom Smp. 92 ° – 93,5 ° C und $[\alpha]_D^{20} = +42° \pm 1°$ (Chloroform, c = 0,973).

Dieses Produkt wird in Methanol gelöst und in Gegenwart von 10 % -Palladium/Kohle hydriert. Nach der Aufarbeitung erhält man das Äthyl-6-deoxy-5-0-methyl-6-(3-methylureido)-a-D-glucofuranosid als Oel vom $[\alpha]_D^{20} = +73° \pm 1°$ (Chloroform, c = 0,330).

Beispiel 2

Eine auf 0 ° C gekühlte Lösung von 4,7 g Äthyl-6-amino-6-deoxy-5-0-methyl-α-D-glucofuranosid in 40 ml Chloroform wird unter Rühren mit einer Lösung von 2,5 g N-Nitroso-methylcarbamylazid in 40 ml Äther tropfenweise versetzt und weitere 3 Stunden bei Raumtemperatur gerührt. Diese Lösung wird zur Trockene eingedampft und der Rückstand in dest. Wasser gelöst. Man extrahiert einmal mit Äther trennt die wässrige Phase ab und lyophilisiert. Man erhält so das Äthyl-6-deoxy-5-0-methyl-6-(3-methyl-3-nitrosoureido)-α-D-glucofuranosid vom Smp. 54–55 ° C und $[\alpha]_D^{20} = +65 \pm 1°$ (Chloroform, c = 0,914).

Beispiel 3

In analoger Weise ausgehend von den entsprechenden Ausgangsstoffen werden die folgenden Verbindungen erhalten:

a) Äthyl-6-[3-(2-chloräthyl)-3-nitrosoureido]-6-deoxy-5-0-methyl-α-D-glucofuranosid

b) Äthyl-6-deoxy-6-deoxy-6-(3-methyl-3-nitrosoureido)-α-D-glucofuranosid

c) 6-Deoxy-1,2-0-isopropyliden-6-(3-methyl-3-nitrosoureido)-α-D-glucofuranose

d) Äthyl-5-0-aethyl-6-deoxy-6-(3-methyl-3-nitrosoureido)-α-a-D-glucofuranosid

e) Äthyl-6-deoxy-6-(3-methyl-3-nitrosoureido)-5-0-propyl-α-D-glucofuranosid

f) Äthyl-5-0-benzyl-6-deoxy-6-(3-methyl-3-nitrosoureido)-α-D-glucofuranosid

g) Methyl-6-deoxy-6-(3-methyl-3-nitrosoureido)-5-0-methyl-α-D-glucofuranosid

h) Äthyl-6-deoxy-2,5-di-0-methyl-6-(3-methyl-3-nitroso-ureido)-α-D-glucofuranosid

i) Äthyl-2-0-acetyl-6-deoxy-5-0-methyl-6-(3-methyl-3-nitrosoureido)-α-D-glucofuranosid

j) 6-Deoxy-1,2-di-0-acetyl-5-0-methyl-6-(3-methyl-3-nitrosoureido)-D-glucofuranose

k) Benzyl-6-deoxy-5-0-methyl-6-(3-methyl-3-nitrosoureido)-D-glucofuranosid.

l) 5-Acetyl-6-deoxy-1,2-0-isopropyliden-6-(3-methyl-3-nitrosoureido)-α-D-glucofuranose

**Patentansprüche**

1. $N_1$-Glucofuranosid-6-yl-$N_3$-nitroso-harnstoffe der Formel I

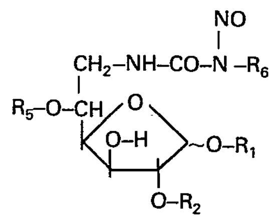

(I)

worin $R_1$, $R_2$ und $R_5$ Wasserstoff, gegebenenfalls durch Hydroxy, Niederalkoxy oder Halogen substituiertes Niederalkyl, gegebenenfalls im aromatischen Teil durch Niederalkyl, Hydroxy, Niederalkoxy, Niederalkylendioxy, Halogen und/oder Trifluormethyl substituiertes Arylniederalkyl, oder Acyl, $R_1$ und $R_2$ zusammen auch Niederalkyliden oder Cycloalkyliden darstellen, und $R_6$ gegebenenfalls durch Hydroxy, Niederalkoxy oder Halogen substituiertes Niederalkyl bedeutet.

2. Verbindungen der Formel I gemäss Anspruch 1, worin $R_1$ und $R_2$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl substituiertes Benzyl, $R_1$ und $R_2$ auch Niederalkyliden oder Cycloalkyliden mit 5–6 Kohlenstoffatomen bedeuten, $R_5$ Wasserstoff, gegebenenfalls durch Hydroxy, Niederalkoxy oder Halogen substituiertes Niederalkyl, gegebenenfalls durch Hydroxy, Niederalkoxy, Halogen oder Trifluormethyl substituiertes Benzyl, Niederalkanoyl oder gegebenenfalls durch Halogen, Niederalkoxy, Hydroxy oder Niederalkanoyloxy substituiertes Benzoyl bedeuten und $R_6$ gegebenenfalls durch Halogen, Hydroxy, oder Niederalkoxy substituiertes Niederalkyl ist.

3. Verbindungen der Formel I gemäss Anspruch 1, worin $R_1$ Niederalkyl und $R_2$ Wasserstoff oder $R_1$ und $R_2$ zusammen Niederalkyliden bedeuten, $R_5$ Wasserstoff, Niederalkyl oder gegebenenfalls durch Halogen, Hydroxy, Niederalkoxy oder Alkyl substituiertes Benzyl und $R_6$ gegebenenfalls mit Chlor substituiertes Niederalkyl darstellen.

4. Verbindungen der Formel I gemäss Anspruch 1, worin $R_1$ Wasserstoff, Methyl, Äthyl oder Propyl und $R_2$ Wasserstoff und $R_1$ und $R_2$ zusammen den Isopropylidenrest bedeuten und $R_5$ Methyl und $R_6$ Methyl oder Chloräthyl darstellen.

5. Aethyl-5-0-methyl-6-deoxy-6-(3-methyl-3-nitrosoureido)-α-D-glucofuranosid.

6. Verbindungen der Formel I gemäss Anspruch 1, als α- oder β-Anomere.

7. Pharmazeutische Präparate enthaltend eine

Verbindung der Formel I gemäss Anspruch 1 zusammen mit inerten Hilfs- und Trägerstoffen.

8. Verbindungen der Formel I gemäss Anspruch 1 zur Verwendung als Arzneimittel.

9. Verwendung von Verbindungen der Formel I gemäss Anspruch 1 zur Herstellung von Arzneimitteln.

10. Verbindungen der Formel I gemäss Anspruch 1 zur Bekämpfung von Tumoren und der Leukämie.

11. Verfahren zur Herstellung von $N_1$-Glucofuranosid-6-yl-$N_3$-nitroso-harnstoffen der Formel

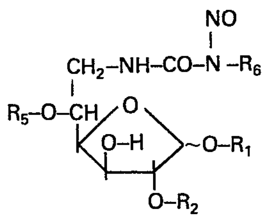

(I)

worin $R_1$, $R_2$ und $R_5$ Wasserstoff, gegebenenfalls durch Hydroxy, Niederalkoxy oder Halogen substituiertes Niederalkyl, gegebenenfalls im aromatischen Teil durch Niederalkyl, Hydroxy, Niederalkoxy, Niederalkylendioxy, Halogen und/oder Trifluormethyl substituiertes Arylniederalkyl oder Acyl, $R_1$ und $R_2$ zusammen auch Alkyliden oder Cycloalkyliden darstellen, und $R_6$ gegebenenfalls durch Hydroxy, Niederalkoxy oder Halogen substituiertes Niederalkyl bedeutet, dadurch gekennzeichnet, dass man

a) eine Verbindung der Formel

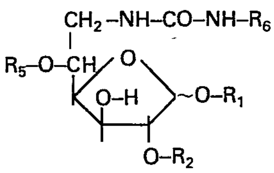

(II)

in an sich bekannter Weise nitrosiert, oder

b) ein Amin der Formel

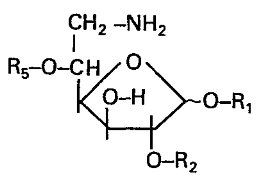

(III)

mit einem reaktionsfähigen Derivat einer N-Nitroso-N-$R_6$-carbaminsäure (IV) kondensiert.

**Revendications**

**1.** $N_1$-glucofuranosid-6-yl-$N_3$-nitrosourées de formule

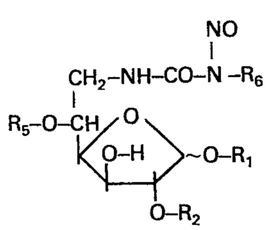

(I)

où $R_1$, $R_2$ et $R_5$ représentent un hydrogène, un alcoyle inférieur éventuellement substitué par un hydroxy, un alcoxy inférieur ou un halogène, un arylalcoyle inférieur éventuellement substitué dans sa fraction aromatique par un alcoyle inférieur, un hydroxy, un alcoxy inférieur, un alcoylidène inférieur-dioxy, un halogène et/ou un trifluorométhyle, ou un acyle, $R_1$ ed $R_2$ représentent également ensemble un alcoylidène inférieur ou un cycloalcoylidène, et $R_6$ représente un alcoyle inférieur éventuellement substitué par un hydroxy, un alcoxy inférieur ou un halogène.

2. Composés de formule I selon la revendication 1, où $R_1$ et $R_2$ représentent un hydrogène, un alcoyle inférieur éventuellement substitué par un hydroxy, un alcoxy inférieur ou un halogène, un benzyle éventuellement substitué par un hydroxy, un alcoxy inférieur, un halogène ou un trifluorométhyle, $R_1$ et $R_2$ présentent également un alcoylidène inférieur ou un cycloalcoylidène ayant 5 ou 6 atomes de carbone, $R_5$ un hydrogène, un alcoyle inférieur éventuellement substitué par un hydroxy, un alcoxy inférieur ou un halogène, un benzyle éventuellement substitué par un hydroxy, un alcoxy inférieur, un halogène ou un trifluorméthyle, un alcanoyle inférieur ou un benzoyle éventuellement substitué par un halogène, un alcoxy inférieur, un hydroxy ou un alcanoyloxy inférieur et $R_6$ est un alcoyle inférieur éventuellement substitué par un halogène, un hydroxy ou un alcoxy inférieur.

3. Composés de formule I selon la revendication 1, oú $R_1$ représente un alcoyle inférieur et $R_2$ un hydrogène ou bien où $R_1$ et $R_2$ représentent ensemble un alcoylidène inférieur, $R_5$ un hydrogène, un alcoyle inférieur ou un benzyle éventuellement substitué par un halogène, un hydroxy, un alcoxy inférieur ou un alcoyle et $R_6$ un alcoyle inférieur éventuellment substitué par un chlore.

4. Composés de formule I selon la revendication 1, où $R_1$ représente un hydrogène, un méthyle, un éthyle ou un propyle et $R_2$ un hydrogène et $R_1$ et $R_2$ représentent ensemble le radical isopropylidène et $R_5$ un méthyle et $R_6$ un méthyle ou un chloroéthyle.

5. Ethyl-5-0-méthyl-6-désoxy-6-(3-méthyl-3-nitrosouréido)-α-D-glucofuranoside.

6. Composés de formule I selon la revendication 1, sous forme d'anomères α ou β.

7. Préparations pharmaceutiques contenant un composé de formule I selon la revendication 1, avec des additifs et supports inertes.

8. Composés de formule I selon la revendication 1, aux fins d'utilisation comme médicaments.

9. Application des composés de formule I selon la revendication 1, à la préparation de médicaments.

10. Composés de formule I selon la revendication 1, pour lutter contre les tumeurs et la leucémie.

11. Procédé de préparation de $N_1$-gluco-furanosid-6-yl-$N_3$-nitrosourées u de formule générale:

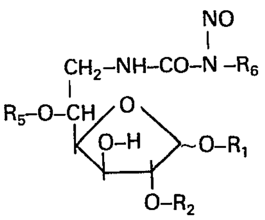

(I)

ou $R_1$, $R_2$ et $R_5$ représentent un hydrogène, un alcoyle inférieur éventuellement substitué par un hydroxy, un alcoxy inférieur ou un halogène, un arylalcoyle inférieur éventuellement substitué dans sa fraction aromatique par un alcoyle inférieur, un hydroxy, un alcoxy inférieur, un alcoylidène inférieur-dioxy, un halogène et/ou un trifluorométhyle, ou un acyle, $R_1$ et $R_2$ représentent également ensemble un alcoylidène ou un cycloalcoylidène, et $R_6$ représente un alcoyle inférieur éventuellement substitué par un hydroxy, un alcoxy inférieur ou un halogène, caractérisé en ce que:

a) on nitrose de façon classique un composé de formule:

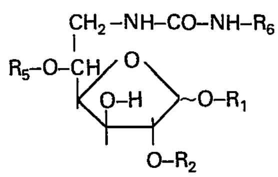

(II)

ou

b) on condense une amine de formule:

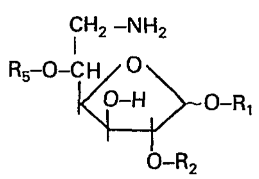

(III)

avec un dérivé réactif d'un acide N-nitrose-N-$R_6$-carbamique (IV).

**Claims**

1. A $N_1$-Glucofuranosid-6-yl-$N_3$-nitroso-urea of the formula I

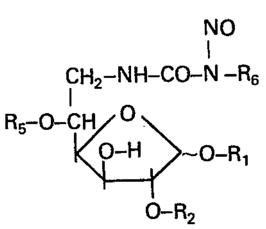

(I)

wherein $R_1$, $R_2$ and $R_5$ are each hydrogen, lower alkyl which is unsubstituted or substituted by hydroxyl, lower alkoxy or halogen, or they are each aryl-lower-alkyl which is unsubstituted or substituted in the aromatic moiety by lower alkyl, hydroxyl, lower alkoxy, lower alkylene-dioxy, halogen and/or trifluoromethyl, or they are each acyl, $R_1$ and $R_2$ together are also lower alkylidene or cycloalkylidene, and $R_6$ is lower alkyl which is unsubstituted or substituted by hydroxyl, lower alkoxy or halogen.

2. A compound of the formula I according to Claim 1, wherein $R_1$ and $R_2$ are each hydrogen, lower alkyl which is unsubstituted or substituted by hydroxyl, lower alkoxy or halogen, or they are each benzyl which is unsubstituted or substituted by hydroxyl, lower alkoxy, halogen or trifluoromethyl, $R_1$ and $R_2$ together are also lower alkylidene or cycloalkylidene each having 5–6 carbon atoms, $R_5$ is hydrogen, lower alkyl which is unsubstituted or substituted by hydroxyl, lower alkoxy or halogen, or it is benzyl which is unsubstituted or substituted by hydroxyl, lower alkoxy, halogen or trifluoromethyl, or it is lower alkanoyl, or benzoyl which is unsubstituted or substituted by halogen, lower alkoxy, hydroxyl or lower alkanoyloxy, and $R_6$ is lower alkyl which is unsubstituted or substituted by halogen, hydroxyl or lower alkoxy.

3. A compound of the formula I according to Claim 1, wherein $R_1$ is lower alkyl and $R_2$ is hydrogen, or $R_1$ and $R_2$ together are lower alkylidene, $R_5$ is hydrogen, lower alkyl, or benzyl which is unsubstituted or substituted by halogen, hydroxyl,lower alkoxy or alkyl, and $R_6$ is lower alkyl which is unsubstituted or substituted by chlorine.

4. A compound of the formula I according to Claim 1, wherein $R_1$ is hydrogen, methyl, ethyl or propyl, $R_2$ is hydrogen, and $R_1$ and $R_2$ together are the isopropylidene group, $R_5$ is methyl, and $R_6$ is methyl or chloroethyl.

5. Ethyl-5-0-methyl-6-deoxy-6-(3-methyl-3-nitrosoureido)-α-D-glucofuranoside.

6. A compound of the formula I according to Claim 1 as an α- or β-anomer.

7. A pharmaceutical preparation containing a compound of the formula I according to Claim 1, together with inert auxiliaries and carriers.

8. A compound of the formula I according to Claim 1 as pharmaceutical active substance.

9. The use of a compound of the formula I according to Claim 1 for producing pharmaceutical preparations.

10. A compound of the formula I according to Claim 1 for combating tumours and leukaemia.

11. A process for producing a $N_1$-glucofuranosid-6-yl-$N_3$-nitroso-urea of the formula

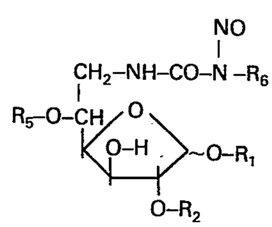

(I)

wherein $R_1$, $R_2$ and $R_5$ are each hydrogen, lower alkyl which is unsubstituted or substituted by hydroxyl, lower alkoxy or halogen, or halogen, or they are each aryl-lower-alkyl which is unsubstituted or substituted in the aromatic moiety by lower alkyl, hydroxyl, lower alkoxy, lower alky-

lenedioxy, halogen and/or trifluoromethyl, or they are each acyl, $R_1$ and $R_2$ together are also alkylidene or cycloalkylidene, and $R_6$ is lower alkyl which is unsubstituted or substituted by hydroxyl, lower alkoxy or halogen, which process comprises:

a) introducing the nitroso group, in a manner known per se, into a compound of the formula

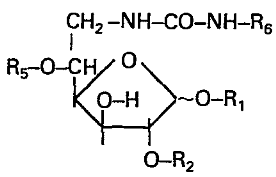

(II)

or

b) condensing an amine of the formula

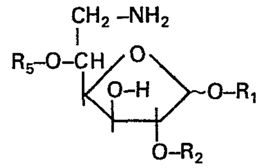

(III)

whith a reactive derivative of an N-nitroso-N-$R_6$-carbamic acid (IV).